(19) Europäisches Patentamt
European Patent Office
Office européen des brevets

(11) Publication number: **0 183 579**
**B1**

(12) # EUROPEAN PATENT SPECIFICATION

(45) Date of publication of patent specification: **06.06.90**

(51) Int. Cl.$^5$: **C 07 C 25/02,** C 07 C 17/156

(21) Application number: **85401990.8**

(22) Date of filing: **14.10.85**

(54) **Method for the synthesis of iodobenzene.**

(30) Priority: **16.10.84 IT 2316984**

(43) Date of publication of application:
**04.06.86 Bulletin 86/23**

(45) Publication of the grant of the patent:
**06.06.90 Bulletin 90/23**

(84) Designated Contracting States:
**BE DE FR GB IT NL**

(56) References cited:
**CHEMICAL ABSTRACTS, vol. 102, no. 19, 13th May 1985, page 590, abstract no. 166447a, Columbus, Ohio, US; & JP - A - 59 219 241**

(73) Proprietor: **MONTEDIPE S.r.l.**
**31, Foro Buonaparte**
**I-20121 Milan (IT)**

(72) Inventor: **Paparatto, Giuseppe**
**7 via Vasari**
**Cinisello Balsamo (Milan) (IT)**
Inventor: **Saetti, Marco**
**73 via Pentapoli**
**I-96010 Priolo (Siracusa) (IT)**

(74) Representative: **Hirsch, Marc-Roger**
**Cabinet Hirsch 34 rue de Bassano**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

The invention concerns a method for the synthesis of iodobenzene by iodination in the gaseous phase of benzene with iodine in the presence of a catalyst having a zeolitic structure.

The synthesis of iodobenzene starting from benzene and iodine is usually carried out in a liquid phase, in the presence of an oxidative agent; preferably the oxidative agent consists of nitric acid (see Japanese patent 58/77830, U.S.S.R. patent 453392 and an article of Datta R. L. and Chatterjee N. R. on the J. Am. Chem. Soc., 39, 437, 1917). Other oxidative agents may also be used none of which, however, has proved, hitherto, to be more efficient and convenient than nitric acid. For instance, the use of iodic acid, sulphur trioxide and hydrogen peroxide has been disclosed (see Butler A. R.: J. Chem. Educ. 36, 508, 1971) as well as the iodination catalyzed by metal halogenides (see Uemura S., Onoe A., Okano M., Bull. Chem. Soc. Jpn., 47, 147, 1974). Japanese patent publication 82/77631 teaches the direct iodination of benzene in a gaseous phase and in the presence of zeolites 13X, but totally excludes the need of adding $O_2$ or any other oxidizing agent. Recently Italian patent publication 19860 A/84, in the name of the Applicant, has described a process for the synthesis of aromatic iodo-substituted compounds starting from alkaline salts of aromatic acids, by reaction with iodine. In all the well known methods, the selectivities to iodine have never been higher than 90%; certain methods utilize expensive oxidative agents and in other methods in which the starting reactants are not readily available. It has now been observed that the synthesis of iodobenzene can be carried out more conveniently according to the reaction:

$$2C_6H_6 + I_2 + 1/2\ O_2 \rightarrow 2C_6H_5I + H_2O \qquad (I)$$

using $O_2$ as oxidative agent and a particular zeolite as catalyst.

In its most general form, the invention relates to a method for the synthesis of iodobenzene by iodination in the gaseous phase, of benzene with iodine, in the presence a catalyst having a zeolitic structure, characterized in that the iodination is an oxidative one, the oxydizing agent being $O_2$, air or other oxygen-containing gas; and in that said catalyst is selected from the group compressing the zeolites the X or Y type in a form different from the acid (H) form. More specifically, a gaseous mixture of benzene, iodine and oxidizing gas is conveyed onto a catalyst based on zeolites of 13X type or of Y type in non-acid form. The catalysts may be used as such or mixed with an appropriate amount of an inert agent, for instance $SiO_2$, acting as binder and/or as carrier.

Earlier European Patent Application 181790 (priority 05.10.87; publication date 21.05.86) discloses an alternative method based on the use of a different kind of catalyst.

The X or Y zeolites have to be used in the form exchanged with monovalent, bivalent or trivalent cations, and in particular with alkaline or rare-earth cations. If use is made of a zeolite exchanged with a mono- or bivalent metal cation, a stabilization of the catalytic activity will be noted; on the contrary, zeolites are used in their acid form, a deterioration of the catalytic activity will be noted. Moreover, in case of zeolites having a low (≤10) $SiO_2/Al_2O_3$ ratio, the acid form cannot be used, since a structure collapse takes place under the reaction conditions and during the optional reactivation.

According to a preferred embodiment of the invention the system consists of a 13X or NaY zeolite.

The catalytic system can also consist of zeolites exchanged with two or more metal cations. For instance, the sodium of the sodic form can be partially exchanged with another metal cation. The same type of catalyst can also be prepared starting from the acid form of the zeolite, carrying out first a partial exchange of the proton with the desired metal cation, using the solution of a hydrosoluble salt thereof. Thereafter, the residual acid sites can be neutralized by means of a diluted solution of NaOH, KOH or $Ca(OH)_2$. When this latter technique is used, a completely exchanged catalyst is obtained and all the Brönsted acid sites, which are responsible for the deterioration of the catalytic activity, are eliminated.

The iodination process can be carried out according to any one of the many known techniques, without departing from the scope and spirit of the invention; by way of example, however, general operating recommendations are given herein-below. A iodine solution in benzene (concentration for 0.5 to 50%, preferably from 5 to 20% by weight) is evaporated and mixed with air in an amount such that the air/$I_2$ molar ratio is at least equal to the stoichiometric ratio and preferably ≥10. The resultant mixture is conveyed to a fixed bed reactor, loaded with the catalyst, the temperature being comprised between 200 and 550°C (preferably between 250 and 450°C) and the space velocity (WHSV) comprised between 0.1 and 100 (preferably between 12 and 20) kg/h of benzene per kg of active part (binder excluded). It is also possible to utilize an inert diluent such as, for instance, nitrogen, helium or steam. The products can then be recovered by cooling the gaseous flow leaving the reactor and by resorting to conventional treatments. In case of distillation, the overhead distilled benzene can be recycled to the iodination reactor. The overall pressure used during the tests is almost always slightly higher than the atmospheric pressure; however, lower or higher pressures may be used. The catalyst maintains its activity over a long period of time, particularly when working, in a gas phase, at 250—450°. However, once the catalytic activity falls below the allowable levels, regeneration starts up. An excellent regeneration consists in activating the catalyst in benzene-air mixtures for a few hours at temperatures comprised between 300 and 600°C. The starting activation of the catalyst is also an important element.

The following examples illustrate the invention without limiting, however, the scope thereof.

Example 1 (NaY)

1 g of a NaY zeolite, produced by Union Carbide Company, was loaded into a quartz microreactor, kept at 400°C by means of a thermostat and continuously fed with a gaseous mixture of benzene, iodine and air, with a benzene/iodine/air molar ratio = 20/1/20. The pressure was slightly higher than the atmospheric pressure and the space velocity (WHSV) was 6 kg/h of benzene/iodine mixture per kg of zeolite. The reaction was carried out for 6 hours and the reaction products were thereafter recovered by condensation. The iodine conversion was 100%, the molar selectivity to iodobenzene 97.5%, to diiodobenzene 2.3% and to others 0.2%.

Data and results are set forth in Table 1, in said table the term "selectivity" means the molar selectivity to benzene.

Example 2 (13X)

Example 1 was repeated, using a 13X zeolite produced by Davison Company as catalyst. Data and results obtained are set forth in Table 1.

Example 3

Example 2 was repeated, lowering the temperature to 325°C; data and results obtained are set forth in Table 1.

Examples 4—15

Examples 2 and 3 were repeated, varying the temperature and the space velocity, as indicated in Table 1, that also shows the results obtained. The 13X zeolite utilized was produced by Union Carbide Company.

Example 16

In order to demonstrate the incidence of space velocity, i.e. of the contact time, example 10 was repeated, increasing the space velocity to 22.8 $h^{-1}$, thereby obtaining the following results, which are obviously less satisfactory:

| | After 1 hour | After 6 hours |
|---|---|---|
| $I_2$ conversion | 97% | 30% |
| Selectivity to: | | |
| Iodobenzene | 96.1% | 94.0% |
| Diiodobenzene | 3.9% | 6.0% |

Examples 17—19

Example 1 was repeated, varying slightly the space velocity and replacing the NaY zeolite with the zeolites indicated in Table 1, the results obtained are also set forth in Table 1.

TABLE 1

| Ex. | Zeolite | T (°C) | WHSV ($h^{-1}$) | Conv. $I_2$ (%) 1 h | Conv. $I_2$ (%) 6 h | Selectivity (%) $C_6H_5I$ 1 h | $C_6H_5I$ 6 h | $C_6H_4I_2$ 1 h | $C_6H_4I_2$ 6 h | others 1 h | others 6 h |
|---|---|---|---|---|---|---|---|---|---|---|---|
| 1 | NaY (a) | 400 | 6 | - | 100 | - | 97.5 | - | 2.3 | - | 0.2 |
| 2 | 13X (b) | 400 | 6 | - | 100 | - | 97.0- | - | 2.8 | - | 0.2 |
| 3 | 13X (b) | 325 | 6 | - | 100 | - | 96.8 | - | 3.0 | - | 0.2 |
| 4 | 13X (a) | 400 | 5.7 | 100 | 100 | 98.1 | 96.3 | 1.9 | 3.7 | - | - |
| 5 | " | 375 | 5.7 | 100 | 100 | 98.0 | 96.8 | 2.0 | 3.2 | - | - |
| 6 | " | 350 | 5.7 | 100 | 100 | 99.0 | 97.3 | 1.0 | 2.7 | - | - |
| 7 | " | 325 | 5.7 | 100 | 100 | 97.4 | 96.4 | 2.6 | 3.6 | - | - |
| 8 | " | 300 | 5.7 | 100 | 100 | 97.0 | 95.1 | 3.0 | 4.9 | - | - |
| 9 | " | 275 | 5.7 | 100 | 100 | 93.6 | 92.1 | 6.4 | 7.9 | - | - |
| 10 | " | 400 | 11.4 | 100 | 100 | 97.7 | 96.2 | 2.3 | 3.8 | - | - |
| 11 | " | 325 | 11.4 | 100 | 97.5 | 95.4 | 95.1 | 4.6 | 4.9 | - | - |
| 12 | " | 270 | 11.4 | 100 | 100 | 96.0 | 93.4 | 4.0 | 6.6 | - | - |
| 13 | " | 250 | 11.4 | 100 | 100 | 94.7 | 91.7 | 5.3 | 9.3 | - | - |
| 14 | " | 230 | 2.8 | 100 | 95.7 | 95.1 | 91.2 | 4.9 | 9.8 | - | - |
| 15 | " | 200 | 2.8 | 100 | 64.0 | 95.0 | 90.0 | 5.0 | 10.0 | - | - |
| 17* | Ca-Y | 400 | 5.7 | 97 | 55 | 98.3 | 98.2 | 1.7 | 1.8 | 0.2 | - |
| 18 | Ca-Na-Y (c) | 400 | 5.7 | 96 | 20 | 98.2 | 98.3 | 1.6 | 1.7 | - | - |
| 19** | Co-Na Y (d) | 400 | 5.7 | 92 | 80 | 96.4 | 95.5 | 3.6 | 4.5 | - | - |

(a) Union Carbide; (b) Davison; (c) $Ca^{++}/Na^{+}$ molar ratio = 65/35; (d) $Co^{++}/Na^{+}$ molar ratio = 70/30
(*) Data survey after 1 h and 3 h
(**) Data survey after 1 h and 4 h.

**Claims**

1. A method for the synthesis of iodobenzene by iodination in the gaseous phase of benzene with iodine, in the presence of a catalyst having a zeolitic structure, characterized in that said iodination is an oxidative one, the oxidizing agent being $O_2$, air or other oxygen containing gases, and in that said catalyst is selected from the group comprising the zeolites of the X or Y type, in a form different from the acid (H) form.

2. A method according to claim 1, wherein the iodination temperature is comprised between 250 and 400°C.

3. A method according to claim 2, wherein the said temperature is comprised between 250 and 400°C.

4. A method according to any one of claims 1 to 3, wherein the oxidizing agent is air and wherein the air/$I_2$ molar ratio is equal to or higher than the stoichiometric ratio.

5. A method according to claim 4, wherein the said molar ratio is ≥ 10.

6. A method according to any one of claims 1 to 5, wherein the concentration of iodine in the benzene feed is comprised between 0.5 and 50% by weight.

7. A method according to claim 6, wherein the said concentration is comprised between 5 and 20% by weight.

8. A method according to any one of claims 1 to 7, wherein the space velocity is comprised between 0.1 and 100 and preferably between 1 and 20 kg/h of benzene per kg of pure zeolite (binder excluded).

9. A method according to any one of claims 1 to 8, wherein said zeolite is a Y zeolite exchanged with cations of alkali metals or rare earth metals, in particular sodium.

10. A method according to claim 1, wherein said zeolite is a X zeolite exchanged with cations of alkali metals or rare earth metals, in particular sodium, as in the case of the 13X zeolite.

11. A method for the synthesis iodobenzene, characterized in that iodine, benzene and air — or another oxygen-containing gas — are brought into contact, in a gaseous phase and at temperatures from 300 to 450°C, with at least one zeolite of 13X or NaY type, the air/$I_2$ molar ratio being higher than or equal to the stoichometric ratio, the space velocity ranging between 1 and 20 kg/h of benzene per kg of pure zeolite — binder excluded — and iodine being fed as a benzenic solution at 5—20% by weight.

**Patentansprüche**

1. Verfahren zur Herstellung von Jodbenzol durch lodierung von Benzol in der Gasphase mit Jod in Gegenward eines Katalysators mit zeolistischer Struktur, dadurch gekennzeichnet, dass die Jodierung eine oxydierende Jodierung ist, wobei Oxydationsmittel $O_2$, Luft oder andere Sauerstoff enthaltende Gase sind, und der Katalysator aus der in anderer als der sauren (H) Form X oder Y Typ Zeolithen umfassenden Gruppe ausgewählt wird.

2. Verfahren nach Anspruch 1, bei dem die Jodierungstemperatur zwischen 200 und 500°C liegt.

3. Verfahren nach Anspruch 2, bei dem die Jodierungstemperatur zwischen 250° und 400° liegt.

4. Verfahren nach einem der Ansprüche 1 bis 3, bei dem das Oxydationsmittel Luft ist und das Molekular-Verhältnis Luft/$J_2$ gleich oder grösser dem stöchiometrischen Verhältnis ist.

5. Verfahren nach Anspruch 4, bei dem das Molekular-Verhältnis gleich oder grösser 10 ist.

6. Verfahren nach einem der Ansprüche 1 bis 5, bei dem die Jodkonzentration im zugeführten Benzol zwischen 0,5 und 50 Gewichtsprozent liegt.

7. Verfahren nach Anspruch 6, bei dem die genannte Konzentration zwischen 5 und 20 Gewichtsprozent liegt.

8. Verfahren nach einem der Ansprüche 1 bis 7, bei dem die Raumgeschwindigkeit zwischen 0,1 und 100, vorzugsweise zwischen 1 und 20 kg/h Benzol pro kg reinen Zeolithen (ohne Bindemittel) liegt.

9. Verfahren nach einem der Ansprüche 1 bis 8, bei dem der Zeolith ein Y-Zeolith der mit Kationen aus Alkalimetallen oder Metallen der Gruppe der seltenen Erden, insbesondere Natrium, ausgetauschten worden ist.

10. Verfahren nach Anspruch 1, bei dem der Zeolith ein X-Zeolith der mit Kationen aus Alkalimetallen oder Metallen der Gruppe der seltenen Erden, insbesondere Natrium, ausgetauschten worden ist, wie es beim 13 X-Zeolithen der Fall ist.

11. Verfahren zur Synthese von Jodbenzol, dadurch gekennzeichnet, dass Jod, Benzol und Luft — oder ein anderes sauerstoffhaltiges Gas -in Gasphasen und bei Temperaturen zwischen 300 und 450°C mit mindestens einem Zeolithen vom 13X oder NaY Typ in Kontakt gebracht werden, wobei das Molekularverhältnis Luft/$J_2$ grösser oder gleich dem Stöchiometrischen ist, wobei die Raumgeschwindigkeit zwischen 1 und 20 kg/h Benzol pro kg reinen Zeolithen — ohne Binder — beträgt und wobei eine 20 Gewichtsprozent Lösung Jod in Benzol zugeführt wird.

**Revendications**

1. Un procédé de synthèse d'iodure de benzène par ioduration en phase gazeuse du benzène avec de l'iode en présence d'un catalysateur ayant une structure zéolitique, caractérisé en ce que l'ioduration est une ioduration oxydante, en ce que l'agent d'oxydation consiste en $O_2$, air ou autres gaz contenant de

l'oxygène et en ce que le catalyseur est choisi dans le groupe des zéolites de type X ou Y autres que sous forme acide (H).

2. Un procédé selon la revendication 1, dans lequel la température d'ioduration est comprise entre 200 et 500°C.

3. Un procédé selon la revendication 1, dans lequel la température d'ioduration est comprise entre 250 et 400°C.

4. Un procédé selon l'une des revendications 1 à 3, dans lequel l'agent d'oxydation est l'air et le rapport moléculaire $Air/I_2$ est égal ou supérieur au rapport stoechiométrique.

5. Un procédé selon la revendication 4, dans lequel le rapport moléculaire est égal ou supérieur à 10.

6. Un procédé selon l'une des revendications 1 à 5, dans lequel la concentration d'iode dans le benzène introduit est comprise entre 0,5 et 50% en poids.

7. Un procédé selon la revendication 6, dans lequel cette concentration est comprise entre 5 et 20% en poids.

8. Un procédé selon l'une des revendications 1 à 7, dans lequel la vitesse spatiale est comprise entre 0,1 et 100, de préférence entre 1 et 20 kg/h benzène par kg de zéolite pure (liant exclus).

9. Un procédé selon l'une des revendications 1 à 8, dans lequel la zéolite est une zéolite de type Y échangée avec des cations de métaux alcalins ou de métaux du groupe des terres rares, notamment du sodium.

10. Un procédé selon la revendication 1, dans lequel le zéolite est une zéolite de type X échangée avec des cations de métaux alcalins ou de métaux du groupe des terres rares, notamment du sodium, ce qui est le cas de la zéolite 13 X.

11. Un procédé de synthèse de iodure de benzène, caractérisé en ce que l'iode, le benzène et l'air — ou un autre gaz riche en oxygène — est mis en contact, en phase gazeuse, avec un moins une zéolite de type 13 X ou NaY à une température comprise entre 300 et 450°C, le rapport moléculaire $air/I_2$ étant égal ou supérieur au rapport stoechiométrique, la vitesse spatiale étant comprise entre 1 et 20 kg/h par kg de zéolite pure (liant exclus) et l'iode étant ajoutée sous forme de solution à 5 à 20% en poids dans le benzène.